# EUROPEAN PATENT APPLICATION

(11) **EP 4 108 092 A1**
(43) Date of publication of application: **28.12.2022**
(21) Application number: 21757473.0
(22) Date of filing: 18.02.2021
(51) Int. Cl.: A23F 3/16, A23F 5/24, A23L 27/00, A23L 27/10, A23L 33/105, A23L 33/16, C02F 1/68, C12G 3/04, A23L 2/00, A23L 2/52, A23L 2/02, A23L 2/38

(54) **LIQUID MINERAL CONCENTRATE COMPOSITION**

(30) Priority: 18.02.2020 JP 2020025724; 18.02.2020 JP 2020025725; 10.03.2020 JP 2020041409; 13.11.2020 JP 2020189878
(71) Applicant: Suntory Holdings Limited, Osaka 530-8203 (JP)
(72) Inventor: OHKURI, Tadahiro, Kawasaki-shi, Kanagawa 211-0067 (JP); YOKOO, Yoshiaki, Kawasaki-shi, Kanagawa 211-0067 (JP); ONUKI, Hitoshi, Tokyo 135-8631 (JP); OSADA, Tomoya, Kawasaki-shi, Kanagawa 211-0067 (JP); FUJIE, Akiko, Kawasaki-shi, Kanagawa 211-0067 (JP); KITA, Ryo, Kawasaki-shi, Kanagawa 211-0067 (JP); TERAMOTO, Yuki, Kawasaki-shi, Kanagawa 211-0067 (JP)
(74) Representative: Vossius & Partner Patentanwälte Rechtsanwälte mbB
(86) International application number: PCT/JP2021/006215
(87) International publication number: WO 2021/167024

(57) **Abstract**

Provided is a liquid mineral concentrate composition that can be added to water, food, drink, or the like to improve the flavor and function thereof. The liquid mineral concentrate composition contains potassium ions the concentration of which is the highest of the metal ions present in the liquid mineral concentrate composition.

## Description

### FIELD

The present invention relates to a liquid mineral concentrate composition that can be added to water, food, drink, or the like to improve the flavor and function thereof.

### BACKGROUND

Against a background of growing health-consciousness and taste-consciousness in recent years, the societal concern to seek safe and good-tasting water has been growing, and mineral water contained in a container such as a PET bottle is a very popular drink all over the world. However, garbage of plastic containers such as PET bottles is posing a serious environmental problem, and accordingly, mineral water that can be served conveniently for household use or the like is under development to replace bottled mineral water.

In addition, what is under development is, for example, a potable water in the form of purified water supplemented with a high concentration of mineral or the like for the purpose of resupplying mineral components that are trace elements necessary for the physiological action of an organism. For example, PTL 1 discloses a potable water containing a high concentration of magnesium, wherein the potable water is produced by mixing purified water with a liquid concentrate containing a large amount of magnesium. PTL 2 discloses a method of producing a drink, wherein mineral components including magnesium and calcium are added to water derived from deep-sea water. However, it is known that divalent metal ions give odd tastes such as bitterness and acridity. Water, food, or drink that contains these minerals at high concentrations has the drawback of being difficult to ingest.

Furthermore, PTL 3 discloses a method of producing mineral water characterized in that immersing natural ore such as granite porphyry, *tenju* stone, or tourmaline in water causes mineral components to be eluted, but the method has drawbacks in that the resulting mineral water contains undesired components such as vanadium that is regarded as harmful if ingested excessively, and in that the efficiency of extraction of minerals is not high. In addition, PTL 4 discloses a method of producing mineral water, wherein chicken dropping charcoal is heated with water for extraction, but chicken dropping charcoal is not suitable as a raw material for use in food applications.

PTL 5 discloses a method of producing mineral water, wherein bamboo charcoal is boiled for extraction, and in addition, PTL 6 discloses a method of producing alkaline water, wherein charcoal is boiled for extraction. However, these methods disclosed in the conventional technologies do not make it possible to extract mineral components efficiently to thereby give a mineral water containing desired mineral components.

### [CITATION LIST]

### [PATENT LITERATURE]

[PTL 1] JP2018-102137A
[PTL 2] JP2008-48742A
[PTL 3] JP2009-72723A
[PTL 4] JP06-31284A
[PTL 5] JP2005-334862A
[PTL 6] JP2001-259659A

### [NON PATENT LITERATURE]

[NPL 1] Abe, I. Production methods of activated carbon, TANSO, 2006, No. 225, 373-381

### SUMMARY

### [TECHNICAL PROBLEM]

An object of the present invention is to provide a liquid mineral concentrate composition that can be added to water, food, drink, or the like to improve the flavor and function thereof.

### [SOLUTION TO PROBLEM]

The present inventors have just recently discovered the use of palm shell activated carbon as a natural material from which minerals can be eluted using pure water, have vigorously made a study on the components of such a liquid mineral concentrate thus produced, and, as a result, have made a surprising discovery that a liquid mineral concentrate composition containing a high concentration of potassium ions give, to purified water having the composition added thereto, a significant buffer capacity in the pH range of from weak alkalinity to weak acidity and besides a mild and less odd flavor.

In other words, a main object of the present invention consists in the following.
[1] A liquid mineral concentrate composition comprising potassium ions the concentration of which is the highest of the metal ions present in the liquid mineral concentrate composition.
[2] The liquid mineral concentrate composition according to 1, wherein the amount of chloride ions contained in the liquid mineral concentrate composition is 50% or less of the potassium ion concentration.
[3] The liquid mineral concentrate composition according to 1 or 2, wherein the amount of calcium ions contained in the liquid mineral concentrate composition is 2.0% or less of the potassium ion concentration.
[4] The liquid mineral concentrate composition according to any one of 1 to 3, wherein the amount of magnesium ions contained in the liquid mineral concentrate composition is 1.0% or less of the potassium ion concentration.
[5] The liquid mineral concentrate composition according to any one of 1 to 4, wherein the amount of sodium contained in the liquid mineral concentrate composition is 5 to 45% of the potassium ion concentration.
[6] The liquid mineral concentrate composition according to any one of 1 to 5, comprising a liquid extract from activated carbon of a plant-derived raw material.
[7] The liquid mineral concentrate composition according to 6, wherein the plant-derived raw material is selected from the following: fruit shells of coconut palms, palms, almonds, walnuts, or plums; woods selected from sawdust, charcoal, resins, and lignin; sawdust ash; bamboos; food residues selected from bagasse, chaff, coffee beans, and molasses; and combinations of these raw materials.
[8] The liquid mineral concentrate composition according to 6, wherein the activated carbon of a plant-derived raw material is palm shell activated carbon.
[9] The liquid mineral concentrate composition according to any one of 1 to 8, having a pH of 7.5 to 10.5.
[10] A water, food, or drink comprising the liquid mineral concentrate composition according to any one of 1 to 9.
[11] The water, food, or drink according to 10, for use in the prevention or improvement of acidification in an organism.

### [ADVANTAGEOUS EFFECTS OF INVENTION]

The present invention makes it possible to improve the flavor and function of water, food, drink, or the like.

### BRIEF DESCRIPTION OF DRAWINGS

FIG. 1 graphs the following: the buffer capacity of each of the aqueous compositions containing different concentrations of added mineral concentrate extracts from palm shell activated carbon; and the buffer capacity of each of the controls (KOH and a commercially available alkaline ionized water).
FIG. 2 graphs the following: the buffer capacity of each of the aqueous compositions that contains an added mineral concentrate extract derived from palm shell activated carbon, and is prepared to have a final potassium concentration of 100 ppm; and the buffer capacity of each of the controls (a purified water and a commercially available alkaline ionized water).
FIG. 3 graphs an organoleptic evaluation of the mild taste of each of the following: the aqueous compositions containing different concentrations of added mineral concentrate extracts derived from palm shell activated carbon; and the control (K₂CO₃).
FIG. 4 graphs an organoleptic evaluation of the odd taste of each of the following: the aqueous compositions containing different concentrations of added mineral concentrate extracts derived from palm shell activated carbon; and the control (K₂CO₃).

### DESCRIPTION OF EMBODIMENTS

The present invention relates to a liquid mineral concentrate composition comprising potassium ions the concentration of which is the highest of the metal ions present in the liquid mineral concentrate composition.

Potassium is one of the minerals necessary for an organism, and the majority of the potassium in an organism is present in the cells. The potassium interacts with a large amount of sodium present in the extracellular fluid, and thus plays an important role in maintaining the osmotic pressure of the cell and holding water in the cell. Potassium, together with sodium, maintains the osmotic pressure of the cell, and besides, serves for functions such as the maintenance of acid-base equilibrium, the innervation, the regulation of the cardiac function and the muscular function, and the regulation of the enzymatic reaction in the cell. In addition, it is known that potassium inhibits the reabsorption of sodium in the kidney, facilitates the excretion into urine, and thus, has the effect of decreasing the blood pressure. As above-mentioned, potassium is a mineral component extremely important for humans, but an excessive amount of potassium ions give odd tastes such as bitterness and acridity. Accordingly, it is preferable that a liquid mineral concentrate composition according to the present invention is prepared in such a manner that, when the liquid mineral concentrate composition is added to water, food, or drink, the lower limit of the potassium concentration or the concentration of the added potassium ions (the potassium concentration of the liquid mineral concentrate composition (ppm) / the dilution ratio) of the water, food, or drink is 20 ppm or more, 25 ppm or more, 30 ppm or more, 35 ppm or more, 45 ppm or more, or 50 ppm or more, and the upper limit of the potassium ion concentration is 600 ppm or less, 595 ppm or less, 590 ppm or less, 585 ppm or less, 580 ppm or less, 575 ppm or less, 570 ppm or less, 565 ppm or less, 560 ppm or less, 555 ppm or less, 550 ppm or less, 545 ppm or less, 540 ppm or less, 535 ppm or less, 530 ppm or less, 525 ppm or less, 520 ppm or less, 515 ppm or less, 510 ppm or less, 505 ppm or less, 500 ppm or less, 495 ppm or less, 490 ppm or less, 485 ppm or less, 480 ppm or less, 475 ppm or less, 470 ppm or less, 465 ppm or less, 460 ppm or less, 455 ppm or less, 450 ppm or less, 445 ppm or less, 440 ppm or less, 435 ppm or less, 430 ppm or less, 425 ppm or less, 420 ppm or less, 415 ppm or less, 410 ppm or less, 405 ppm or less, 400 ppm or less, 395 ppm or less, 390 ppm or less, 385 ppm or less, 380 ppm or less, 375 ppm or less, 370 ppm or less, 365 ppm or less, 360 ppm or less, 355 ppm or less, 350 ppm or less, 345 ppm or less, 340 ppm or less, 335 ppm or less, 330 ppm or less, 325 ppm or less, 320 ppm or less, 315 ppm or less, 310 ppm or less, 305 ppm or less, 300 ppm or less, 295 ppm or less, 290 ppm or less, 285 ppm or less, 280 ppm or less, 275 ppm or less, 270 ppm or less, 265 ppm or less, 260 ppm or less, 255 ppm or less, 250 ppm or less, 245 ppm or less, 240 ppm or less, 235 ppm or less, 230 ppm or less, 225 ppm or less, 220 ppm or less, 215 ppm or less, 210 ppm or less, 205 ppm or less, or 200 ppm or less. A liquid mineral concentrate composition according to the present invention can be prepared in such a manner that, when the liquid mineral concentrate composition is added to water, food, or drink, the potassium concentration or the concentration of the added potassium ions (the potassium concentration of the liquid mineral concentrate composition (ppm) / the dilution ratio) of the water, food, or drink is, for example, 50 to 200 ppm, 50 to 190 ppm, 50 to 180 ppm, 50 to 170 ppm, 50 to 160 ppm, 50 to 150 ppm, 50 to 140 ppm, 50 to 130 ppm, 50 to 120 ppm, 50 to 110 ppm, 50 to 100 ppm, 50 to 90 ppm, 50 to 80 ppm, 50 to 70 ppm, 50 to 60 ppm, 60 to 200 ppm, 60 to 190 ppm, 60 to 180 ppm, 60 to 170 ppm, 60 to 160 ppm, 60 to 150 ppm, 60 to 140 ppm, 60 to 130 ppm, 60 to 120 ppm, 60 to 110 ppm, 60 to 100 ppm, 60 to 90 ppm, 60 to 80 ppm, 60 to 70 ppm, 70 to 200 ppm, 70 to 190 ppm, 70 to 180 ppm, 70 to 170 ppm, 70 to 160 ppm, 70 to 150 ppm, 70 to 140 ppm, 70 to 130 ppm, 70 to 120 ppm, 70 to 110 ppm, 70 to 100 ppm, 70 to 90 ppm, 70 to 80 ppm, 80 to 200 ppm, 80 to 190 ppm, 80 to 180 ppm, 80 to 170 ppm, 80 to 160 ppm, 80 to 150 ppm, 80 to 140 ppm, 80 to 130 ppm, 80 to 120 ppm, 80 to 110 ppm, 80 to 100 ppm, 80 to 90 ppm, 90 to 200 ppm, 90 to 190 ppm, 90 to 180 ppm, 90 to 170 ppm, 90 to 160 ppm, 90 to 150 ppm, 90 to 140 ppm, 90 to 130 ppm, 90 to 120 ppm, 90 to 110 ppm, 90 to 100 ppm, 100 to 200 ppm, 100 to 190 ppm, 100 to 180 ppm, 100 to 170 ppm, 100 to 160 ppm, 100 to 150 ppm, 100 to 140 ppm, 100 to 130 ppm, 100 to 120 ppm, 100 to 110 ppm, 110 to 200 ppm, 110 to 190 ppm, 110 to 180 ppm, 110 to 170 ppm, 110 to 160 ppm, 110 to 150 ppm, 110 to 140 ppm, 110 to 130 ppm, 110 to 120 ppm, 120 to 200 ppm, 120 to 190 ppm, 120 to 180 ppm, 120 to 170 ppm, 120 to 160 ppm, 120 to 150 ppm, 120 to 140 ppm, 120 to 130 ppm, 130 to 200 ppm, 130 to 190 ppm, 130 to 180 ppm, 130 to 170 ppm, 130 to 160 ppm, 130 to 150 ppm, 130 to 140 ppm, 140 to 200 ppm, 140 to 190 ppm, 140 to 180 ppm, 140 to 170 ppm, 140 to 160 ppm, 140 to 150 ppm, 150 to 200 ppm, 150 to 190 ppm, 150 to 180 ppm, 150 to 170 ppm, 150 to 160 ppm, 160 to 200 ppm, 160 to 190 ppm, 160 to 180 ppm, 160 to 170 ppm, 170 to 200 ppm, 170 to 190 ppm, 170 to 180 ppm, 180 to 200 ppm, 180 to 190 ppm, or 190 to 200 ppm.

Naturally-occurring water contains a given amount of chloride ions, and many of the ions are derived from natural soil or sea water. Chloride ions, if present at 250 to 400 mg/l or more, give a taste salty for a taste-sensitive person, and can impair the taste, and hence, the amount of chloride ions contained in a liquid mineral concentrate composition according to the present invention is preferably as small as possible. A liquid mineral concentrate composition according to the present invention can be prepared in such a manner that, when the liquid mineral concentrate composition is added to water, food, or drink, the amount of chloride ions contained in the water, food, or drink is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, or 1% or less of the above-mentioned potassium ion concentration. The amount of chloride ions contained in a liquid mineral concentrate composition according to the present invention is, for example, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31% or less, 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, or 1% or less of the potassium ion concentration.

It is known that calcium together with phosphorus, in the form of hydroxyapatite, is skeletogenous in an organism, and participates in muscle contraction. It is known that magnesium participates in osteogenesis, odontogenesis, many intracorporeal enzymatic reactions, and energy production in an organism. In addition, it is known that the amount of calcium ions and magnesium ions contained in water influences the taste of the water. The index (hardness) as the total amount of calcium and magnesium contained in the minerals contained in water is smaller for what is termed soft water than a given level, and larger for what is termed hard water. In general, more of the mineral water produced domestically in Japan is soft water, and more of the mineral water produced in Europe is hard water. According to the criteria stipulated by WHO, the U.S. hardness (mg/1) in terms of calcium carbonate converted from the amount of these salts is 0 to 60 for what is termed soft water, 120 to 180 for what is termed hard water, and 180 or more for what is termed very hard water. In general, water having a suitable hardness (10 to 100 mg/1) is regarded as good-tasting. Water containing a higher amount of magnesium in particular is bitterer, and more difficult to drink. In addition, a higher hardness not only influences the taste of water, but also stimulates the stomach and intestines, causes diarrhea or the like, and hence, is not preferable. Accordingly, it is preferable that a liquid mineral concentrate composition according to the present invention is prepared in such a manner that, when the liquid mineral concentrate composition is added to water, food, or drink, the amount of calcium ions in the water, food, or drink is, for example, 30% or less, 29% or less, 28% or less, 27% or less, 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, 10% or less, 9% or less, 8% or less, 7% or less, 6% or less, 5% or less, 4% or less, 3% or less, 2% or less, or 1% or less of the potassium ion concentration, and the amount of magnesium ions in the water, food, or drink is, for example, 15% or less, 14% or less, 13% or less, 12% or less, 11% or less, 10% or less, 9% or less, 8% or less, 7% or less, 6% or less, 5% or less, 4% or less, 3% or less, 2% or less, or 1% or less of the potassium ion concentration. The amount of calcium ions contained in a liquid mineral concentrate composition according to the present invention is, for example, 2.0% or less, 1.9% or less, 1.8% or less, 1.7% or less, 1.6% or less, 1.5% or less, 1.4% or less, 1.3% or less, 1.2% or less, 1.1% or less, 1.0% or less, 0.9% or less, 0.8% or less, 0.7% or less, 0.6% or less, 0.5% or less, 0.4% or less, 0.3% or less, 0.2% or less, 0.1% or less, 0.09% or less, 0.08% or less, 0.07% or less, 0.06% or less, 0.05% or less, 0.04% or less, 0.03% or less, 0.02% or less, or 0.01% or less of the potassium ion concentration. In addition, the amount of magnesium ions contained in a liquid mineral concentrate composition according to the present invention is, for example, 1.0% or less, 0.9% or less, 0.8% or less, 0.7% or less, 0.6% or less, 0.5% or less, 0.4% or less, 0.3% or less, 0.2% or less, 0.1% or less, 0.09% or less, 0.08% or less, 0.07% or less, 0.06% or less, 0.05% or less, 0.04% or less, 0.03% or less, 0.02% or less, or 0.01% or less of the potassium ion concentration.

Sodium holds water in an organism, maintaining the amount of the extracellular fluid and the amount of the circulating blood, and regulating the blood pressure. It is known that the ingestion of a given amount of sodium ions is good for effective intracorporeal rehydration, and efficacious as the countermeasures particularly against heat stroke or the like. However, excessive ingestion of sodium increases the amount of such a liquid, and thus, will undesirably raise the blood pressure, and cause dropsy. In addition, a higher amount of sodium ions give a saltier taste and a slimier feeling, and impairs the refreshing taste of a drink in some cases. Accordingly, it is preferable that a liquid mineral concentrate composition according to the present invention is prepared in such a manner that, when the liquid mineral concentrate composition is added to water, food, or drink, the sodium ion concentration of the water, food, or drink is, for example, 10 to 50%, 10 to 45%, 10 to 40%, 10 to 35%, 10 to 30%, 10 to 25%, 10 to 20%, 10 to 15%, 15 to 50%, 15 to 45%, 15 to 40%, 15 to 35%, 15 to 30%, 15 to 25%, 15 to 20%, 20 to 50%, 20 to 45%, 20 to 40%, 20 to 35%, 20 to 30%, 20 to 25%, 25 to 50%, 25 to 45%, 25 to 40%, 25 to 35%, 25 to 30%, 30 to 50%, 30 to 45%, 30 to 40%, 30 to 35%, 35 to 50%, 35 to 45%, 35 to 40%, 40 to 50%, 40 to 45%, or 45 to 50% of the potassium ion concentration. The amount of sodium contained in the liquid mineral concentrate composition is, for example, 5 to 45%, 5 to 40%, 5 to 35%, 5 to 30%, 5 to 25%, 5 to 20%, 5 to 15%, 5 to 10%, 10 to 45%, 10 to 40%, 10 to 35%, 10 to 30%, 10 to 25%, 10 to 20%, 10 to 15%, 15 to 45%, 15 to 40%, 15 to 35%, 15 to 30%, 15 to 25%, 15 to 20%, 20 to 45%, 20 to 40%, 20 to 35%, 20 to 30%, 20 to 25%, 25 to 50%, 25 to 45%, 25 to 40%, 25 to 35%, 25 to 30%, 30 to 45%, 30 to 40%, 30 to 35%, 35 to 45%, 35 to 40%, or 40 to 45% of the potassium ion concentration.

Adding a liquid mineral concentrate composition according to the present invention to water, food, or drink makes it possible to produce weak alkaline water, food, or drink. For example, water containing a liquid mineral concentrate composition according to the present invention may typically have a pH of 7.5 to 10.5, 7.5 to 10.0, 7.5 to 9.5, 7.5 to 9.0, 7.5 to 8.5, 7.5 to 8.0, 8.0 to 10.5, 8.0 to 10.0, 8.0 to 9.5, 8.0 to 9.0, 8.0 to 8.5, 8.5 to 10.5, 8.5 to 10.0, 8.5 to 9.5, 8.5 to 9.0, 9.0 to 10.5, 9.0 to 10.0, 9.0 to 9.5, 9.5 to 10.5, 9.5 to 10.0, or 10.0 to 10.5. In addition, water containing a liquid mineral concentrate composition according to the present invention has a buffer capacity, and preferably has a significant buffer capacity in the pH range of from weak alkalinity to weak acidity. For example, the water containing a liquid mineral concentrate composition according to the present invention has a buffer capacity of 1.5 or more, 1.6 or more, 1.7 or more, 1.8 or more, 1.9 or more, 2.0 or more, 2.1 or more, 2.2 or more, 2.3 or more, 2.4 or more, 2.5 or more, 2.6 or more, 2.7 or more, 2.8 or more, 2.9 or more, 3.0 or more, 3.5 or more, 4.0 or more, 4.5 or more, 5.0 or more, 5.5 or more, 6.0 or more, 6.5 or more, 7.0 or more, 7.5 or more, 8.0 or more, 8.5 or more, 9.0 or more, 9.5 or more, 10.0 or more, 10.5 or more, 11.0 or more, or 11.5 or more, for example, wherein the buffer capacity is defined as a ratio (B)/(A), assuming that the amount of 0.1 M hydrochloric acid solution with which 100 g of a sodium hydroxide solution adjusted to a pH of 9.2 is titrated from a pH of 9.2 to a pH of 3.0 is (A) mL, and that the amount of 0.1 M hydrochloric acid solution with which the water containing the liquid mineral concentrate composition according to the present invention is titrated from a pH of 9.2 to a pH of 3.0 is (B) mL. Such pH characteristics prevent or improve acidification in an organism, and hence are useful. Accordingly, adding a liquid mineral concentrate composition according to the present invention to water (for example, purified water), food, or drink makes it possible, for example, to prevent a tooth from acid erosion due to acidification in the oral cavity after a meal, and to improve gastrointestinal symptoms such as hyperchlorhydria or abnormal enteric fermentation due to acidification in the stomach and intestines.

A liquid mineral concentrate composition according to the present invention may comprise a liquid extract from activated carbon of a plant-derived raw material. Activated carbon is a porous substance composed largely of carbon and additionally of oxygen, hydrogen, calcium, and the like, has a large surface area per volume, and thus, has the property of adsorbing many substances, and hence, is widely produced industrially from the early twentieth century to now. In general, activated carbon is produced by generating (activating) the nm-level micropores inside a carbon material serving as a raw material. Methods of producing activated carbon is generally classified into the following: a gas activation method in which a raw material is carbonized, and then, the resulting product is activated at high temperature using an activation gas such as water vapor or carbon dioxide; and a chemical agent activation method in which a chemical agent such as zinc chloride or phosphoric acid is added to a raw material, and the, the resulting mixture is carbonized and activated at once under heating in an inert gas atmosphere (NPL 1). Activated carbon to be used in the present invention can be produced by one of the above-mentioned gas activation method and the chemical agent activation method, using a plant-derived raw material as a carbon material.

A raw material for activated carbon to be used in the present invention is subject to no particular limitation as long as the raw material is plant-derived. Examples of such a raw material include: fruit shells (coconut palms, palms, almonds, walnuts, and plums); woods (sawdust, charcoal, resins, and lignin); sawdust ash (carbide of sawdust); bamboos; food residues (bagasse, chaff, coffee beans, and molasses); wastes (pulp mill waste liquids and construction and demolition wastes); and the like. Such a raw material is typically selected from palm shells, sawdust, bamboos, and combinations thereof, and is suitably palm shells. A palm shell means a hard part-called a shell-in a fruit of a coconut palm or a palm.

The shape of activated carbon to be used in the present invention is subject to no particular limitation. Examples of the activated carbon include powdery activated carbon, particulate activated carbon (crushed carbon, granular carbon, and molded carbon), fibrous activated carbon, specially molded activated carbon, and the like.

A step of extracting minerals from activated carbon of a plant-derived raw material using an aqueous solvent is performed by bringing activated carbon of a plant-derived raw material in contact with an aqueous solvent, and eluting minerals from activated carbon of a plant-derived raw material. Such a step is subject to no particular limitation as long as the step makes it possible to elute minerals from activated carbon of a plant-derived raw material. For example, such a step can be performed by immersing activated carbon of a plant-derived raw material in an aqueous solvent, or allowing an aqueous solvent to pass through a column packed with activated carbon of a plant-derived raw material. In cases where activated carbon of a plant-derived raw material is immersed in an aqueous solvent, the aqueous solvent may be stirred to increase the efficiency of extraction. To remove impurities from a liquid given by extracting minerals from the activated carbon of a plant-derived raw material using an aqueous solvent, a method of producing a liquid mineral extract may further include a step of centrifuging the resulting liquid extract, a step of filtrating the liquid extract, and/or the like.

An aqueous solvent to be used in a step of extracting minerals from activated carbon of a plant-derived raw material using an aqueous solvent basically refers to an aqueous solvent other than an HCl solution. Such a solvent is typically a water solvent, and is particularly preferably pure water. Pure water means high-purity water containing no or few impurities such as salts, residual chlorine, insoluble microparticles, organic substances, and nonelectrolytic gas. Pure water encompasses RO water (water passed through a reverse osmosis membrane), deionized water (water from which ions have been removed with an ion exchange resin or the like), distilled water (water distilled with a distiller), and the like, which differ in the method of removing impurities. Pure water contains no mineral component, and hence, does not exhibit any effect of resupplying minerals.

The extraction temperature is subject to no particular limitation as long as the temperature makes it possible to extract minerals from activated carbon of a plant-derived raw material using an aqueous solvent. The step of extracting minerals from activated carbon of a plant-derived raw material using an aqueous solvent can be performed at a temperature of 5°C or more, 10°C or more, 15°C or more, 20°C or more, 25°C or more, 30°C or more, 35°C or more, 40°C or more, 45°C or more, 50°C or more, 55°C or more, 60°C or more, 65°C or more, 70°C or more, 75°C or more, 80°C or more, 85°C or more, 90°C or more, or 95°C or more, and is performed, for example at a temperature of 5 to 95°C, 5 to 90°C, 5 to 85°C, 5 to 80°C, 5 to 75°C, 5 to 70°C, 5 to 65°C, 5 to 60°C, 5 to 55°C, 5 to 50°C, 5 to 45°C, 5 to 40°C, 5 to 35°C, 5 to 30°C, 5 to 25°C, 5 to 20°C, 5 to 15°C, 5 to 10°C, 10 to 95°C, 10 to 90°C, 10 to 85°C, 10 to 80°C, 10 to 75°C, 10 to 70°C, 10 to 65°C, 10 to 60°C, 10 to 55°C, 10 to 50°C, 10 to 45°C, 10 to 40°C, 10 to 35°C, 10 to 30°C, 10 to 25°C, 10 to 20°C, 10 to 15°C, 15 to 95°C, 15 to 90°C, 15 to 85°C, 15 to 80°C, 15 to 75°C, 15 to 70°C, 15 to 65°C, 15 to 60°C, 15 to 55°C, 15 to 50°C, 15 to 45°C, 15 to 40°C, 15 to 35°C, 15 to 30°C, 15 to 25°C, 15 to 20°C, 20 to 95°C, 20 to 90°C, 20 to 85°C, 20 to 80°C, 20 to 75°C, 20 to 70°C, 20 to 65°C, 20 to 60°C, 20 to 55°C, 20 to 50°C, 20 to 45°C, 20 to 40°C, 20 to 35°C, 20 to 30°C, 20 to 25°C, 25 to 95°C, 25 to 90°C, 25 to 85°C, 25 to 80°C, 25 to 75°C, 25 to 70°C, 25 to 65°C, 25 to 60°C, 25 to 55°C, 25 to 50°C, 25 to 45°C, 25 to 40°C, 25 to 35°C, 25 to 30°C, 30 to 95°C, 30 to 90°C, 30 to 85°C, 30 to 80°C, 30 to 75°C, 30 to 70°C, 30 to 65°C, 30 to 60°C, 30 to 55°C, 30 to 50°C, 30 to 45°C, 30 to 40°C, 30 to 35°C, 35 to 95°C, 35 to 90°C, 35 to 85°C, 35 to 80°C, 35 to 75°C, 35 to 70°C, 35 to 65°C, 35 to 60°C, 35 to 55°C, 35 to 50°C, 35 to 45°C, 35 to 40°C, 40 to 95°C, 40 to 90°C, 40 to 85°C, 40 to 80°C, 40 to 75°C, 40 to 70°C, 40 to 65°C, 40 to 60°C, 40 to 55°C, 40 to 50°C, 40 to 45°C, 45 to 95°C, 45 to 90°C, 45 to 85°C, 45 to 80°C, 45 to 75°C, 45 to 70°C, 45 to 65°C, 45 to 60°C, 45 to 55°C, 45 to 50°C, 50 to 95°C, 50 to 90°C, 50 to 85°C, 50 to 80°C, 50 to 75°C, 50 to 70°C, 50 to 65°C, 50 to 60°C, 50 to 55°C, 55 to 95°C, 55 to 90°C, 55 to 85°C, 55 to 80°C, 55 to 75°C, 55 to 70°C, 55 to 65°C, 55 to 60°C, 60 to 95°C, 60 to 90°C, 60 to 85°C, 60 to 80°C, 60 to 75°C, 60 to 70°C, 60 to 65°C, 65 to 95°C, 65 to 90°C, 65 to 85°C, 65 to 80°C, 65 to 75°C, 65 to 70°C, 70 to 95°C, 70 to 90°C, 70 to 85°C, 70 to 80°C, 70 to 75°C, 75 to 95°C, 75 to 90°C, 75 to 85°C, 75 to 80°C, 80 to 95°C, 80 to 90°C, 80 to 85°C, 85 to 95°C, 85 to 90°C, or 90 to 95°C.

The extraction time is subject to no particular limitation as long as the time makes it possible to extract minerals from activated carbon of a plant-derived raw material using an aqueous solvent. The step of extracting minerals from activated carbon of a plant-derived raw material using an aqueous solvent can be performed for 5 minutes or more, 10 minutes or more, 15 minutes or more, 20 minutes or more, 25 minutes or more, 30 minutes or more, 35 minutes or more, 40 minutes or more, 45 minutes or more, 50 minutes or more, 55 minutes or more, 60 minutes or more, 65 minutes or more, 70 minutes or more, 75 minutes or more, or 80 minutes or more, and is performed, for example, for 5 to 80 minutes, 5 to 75 minutes, 5 to 70 minutes, 5 to 65 minutes, 5 to 60 minutes, 5 to 55 minutes, 5 to 50 minutes, 5 to 45 minutes, 5 to 40 minutes, 5 to 35 minutes, 5 to 30 minutes, 5 to 25 minutes, 5 to 20 minutes, 5 to 15 minutes, 5 to 10 minutes, 10 to 80 minutes, 10 to 75 minutes, 10 to 70 minutes, 10 to 65 minutes, 10 to 60 minutes, 10 to 55 minutes, 10 to 50 minutes, 10 to 45 minutes, 10 to 40 minutes, 10 to 35 minutes, 10 to 30 minutes, 10 to 25 minutes, 10 to 20 minutes, 10 to 15 minutes, 15 to 80 minutes, 15 to 75 minutes, 15 to 70 minutes, 15 to 65 minutes, 15 to 60 minutes, 15 to 55 minutes, 15 to 50 minutes, 15 to 45 minutes, 15 to 40 minutes, 15 to 35 minutes, 15 to 30 minutes, 15 to 25 minutes, 15 to 20 minutes, 20 to 80 minutes, 20 to 75 minutes, 20 to 70 minutes, 20 to 65 minutes, 20 to 60 minutes, 20 to 55 minutes, 20 to 50 minutes, 20 to 45 minutes, 20 to 40 minutes, 20 to 35 minutes, 20 to 30 minutes, 20 to 25 minutes, 25 to 80 minutes, 25 to 75 minutes, 25 to 70 minutes, 25 to 65 minutes, 25 to 60 minutes, 25 to 55 minutes, 25 to 50 minutes, 25 to 45 minutes, 25 to 40 minutes, 25 to 35 minutes, 25 to 30 minutes, 30 to 80 minutes, 30 to 75 minutes, 30 to 70 minutes, 30 to 65 minutes, 30 to 60 minutes, 30 to 55 minutes, 30 to 50 minutes, 30 to 45 minutes, 30 to 40 minutes, 30 to 35 minutes, 35 to 80 minutes, 35 to 75 minutes, 35 to 70 minutes, 35 to 65 minutes, 35 to 60 minutes, 35 to 55 minutes, 35 to 50 minutes, 35 to 45 minutes, 35 to 40 minutes, 40 to 80 minutes, 40 to 75 minutes, 40 to 70 minutes, 40 to 65 minutes, 40 to 60 minutes, 40 to 55 minutes, 40 to 50 minutes, 40 to 45 minutes, 45 to 80 minutes, 45 to 75 minutes, 45 to 70 minutes, 45 to 65 minutes, 45 to 60 minutes, 45 to 55 minutes, 45 to 50 minutes, 50 to 80 minutes, 50 to 75 minutes, 50 to 70 minutes, 50 to 65 minutes, 50 to 60 minutes, 50 to 55 minutes, 55 to 80 minutes, 55 to 75 minutes, 55 to 70 minutes, 55 to 65 minutes, 55 to 60 minutes, 60 to 80 minutes, 60 to 75 minutes, 60 to 70 minutes, 60 to 65 minutes, 65 to 80 minutes, 65 to 75 minutes, 65 to 70 minutes, 70 to 80 minutes, 70 to 75 minutes, or 75 to 80 minutes.

The liquid mineral extract given in this manner is concentrated, whereby a liquid mineral concentrate composition can be given.

A step of concentrating a liquid mineral extract can be performed using a method known in the art. Examples of such a method include boiling concentration, vacuum concentration, freeze concentration, membrane concentration, ultrasonic humidification separation, and the like. Concentrating a liquid mineral extract makes it possible to obtain a liquid mineral concentrate composition containing a desired mineral such as high-concentration potassium almost without changing the composition of the liquid.

After the step of concentrating the liquid mineral extract, the resulting liquid mineral concentrate composition is preferably stored under refrigeration and filtrated under cooling. The cooling temperature is typically adjusted to 0 to 15°C, preferably 3 to 10°C, 3 to 9°C, 3 to 8°C, 3 to 7°C, or 3 to 6°C. In addition, the pH of the liquid mineral concentrate composition is preferably adjusted before such storage under refrigeration and filtration under cooling. The liquid mineral concentrate composition is adjusted so as to have a pH of, for example, 7.5 to 10.5, 7.5 to 10.0, 7.5 to 9.5, 7.5 to 9.0, 7.5 to 8.5, 7.5 to 8.0, 8.0 to 10.5, 8.0 to 10.0, 8.0 to 9.5, 8.0 to 9.0, 8.0 to 8.5, 8.5 to 10.5, 8.5 to 10.0, 8.5 to 9.5, 8.5 to 9.0, 9.0 to 10.5, 9.0 to 10.0, 9.0 to 9.5, 9.5 to 10.5, 9.5 to 10.0, or 10.0 to 10.5. Performing such a treatment makes it possible to obtain a liquid mineral concentrate composition having high transparency and a significantly decreased amount of suspended matter and precipitate.

A container for providing a liquid mineral concentrate composition according to the present invention is not limited to any particular form. Examples of the form include: metal containers (cans); resin containers such as of a dropping type, spray type, dropper type, or lotion bottle type; paper containers (including paper containers with a gable top); PET bottles; pouch containers; glass bottles; airless containers; portion containers; antiseptic-free (PF) eyedrop containers; stick packs; small pump containers; large pump containers; portion cup containers; inner package-containing bottles; single-use plastic containers; water-soluble film containers; and the like. It is also possible that a liquid mineral concentrate composition according to the present invention is automatically mixed with tap water or purified water to provide weak alkaline mineral water continuously.

A liquid mineral concentrate composition according to the present invention can be added to water, food, drink, or the like to improve the flavor and function thereof. Examples of conceivable applications of a liquid mineral concentrate composition according to the present invention include the following:
- to be added dropwise to tap water, purified water, or pure water to make mineral water;
- to be added dropwise to an alcohol such as whiskey to improve the flavor thereof;
- to be added dropwise to mineral water to make water to be served as a drink together with wine or the like;
- to be added dropwise to an extract, powder, or drink of coffee liquid, coffee drink, tea infusion, tea drink, or the like to make the flavor mild;
- to be added dropwise to extract water of coffee beans or tea leaves to increase the efficiency of extraction;
- to be added dropwise to water for rice steaming to improve the flavor of rice right after steaming;
- to be added dropwise to a liquid such as water, for use in the improvement of an unpleasant symptom in the stomach and intestines of a person having a weak stomach and intestines and a person with hyperchlorhydria;
- to be added dropwise to a liquid such as water, for use in the improvement of the blood pressure of a person having high blood pressure;
- to be automatically mixed with tap water and purified water to serve as potable water or water having a bactericidal effect for hand-washing; and
- to be added dropwise to a plant, which, with a liquid mineral concentrate composition according to the present invention, can be used in the form of a mineral nutritional supplement, as well as to be added to water, food, drink, or the like.

Below, the present invention will be described in further detail with reference to Examples. However, the present invention is not limited to the below-mentioned Examples, and can be carried out with a suitable change.

### EXAMPLES

### <Example 1: production of liquid mineral extract from palm shell activated carbon>

Into a 1 L Erlenmeyer flask, 30 g of palm shell activated carbon ("TAIKO CW Type", not cleaned, manufactured by Futamura Chemical Co., Ltd.) and 400 g of distilled water heated to 90°C were introduced, and the resulting mixture was stirred with a stirring bar under heating at 90°C at 100 rpm for 15 minutes. The resulting suspension was filtrated with suction through a polyester mesh of 500 (25 µm), and the resulting filtrate was centrifuged at 3000 rpm for 10 minutes. After the centrifugation, the resulting supernatant was filtrated with suction through a paper filter to give a liquid mineral extract.

### <Example 2: comparison of activated carbon>

A liquid mineral extract was produced by the same method as in Example 1 except that the palm shell activated carbon was changed to KURARAY COAL (registered trademark) GG (not cleaned, manufactured by Kuraray Co., Ltd.).

### <Examples 3 to 6: comparison of extraction time>

Liquid mineral extracts were produced by the same method as in Example 1 except that the extraction time was changed to 10, 20, 40, and 80 minutes.

### <Examples 7 to 9: comparison of distilled water amount and of extraction time>

Liquid mineral extracts were produced by the same method as in Example 1 except that the amount of distilled water was changed to 130, 200, and 400 g, and that the extraction time was changed to 5 minutes.

### <Examples 10 to 12: comparison of extraction temperature and of extraction time>

Liquid mineral extracts were produced by the same method as in Example 1 except that the extraction temperature was changed to 30, 60, and 90°C, and that the extraction time was changed to 5 minutes.

The liquid mineral extracts produced in Examples 1 to 12 were analyzed in accordance with the following method.

### <ICP analysis of metal>

An ICP atomic emission spectrometer iCAP6500Duo (manufactured by Thermo Fisher Scientific Inc.) was used. A general-purpose liquid mixture XSTC-622B for ICP was diluted to prepare a 4-point calibration curve based on 0, 0.1, 0.5, and 1.0 mg/L. The sample was diluted with dilute nitric acid so as to fall within the range of the calibration curve, and subjected to ICP measurement.

### <IC analysis of Cl⁻ and SO₄²⁻>

An ion chromatograph system ICS-5000K (manufactured by Nippon Dionex K.K.) was used. The columns used were Dionex Ion Pac AG20 and Dionex Ion Pac AS20. As an eluent, an aqueous solution of 5 mmol/L potassium hydroxide was used for the section from 0 to 11 minutes, 13 mmol/L for the section from 13 to 18 minutes, and 45 mmol/L for the section from 20 to 30 minutes for elution at a flow rate of 0.25 mL/minute. A negative ion-containing standard solution mixture 1 (containing seven species of ions including Cl⁻ at 20 mg/L and SO₄²⁻ at 100 mg/L, manufactured by Fujifilm Wako Pure Chemical Corporation) was diluted to prepare a 5-point calibration curve based on 0, 0.1, 0.2, 0.4, and 1.0 mg/L for Cl⁻ and a 5-point calibration curve based on 0, 0.5, 1.0, 2.0, and 5.0 mg/L for SO₄²⁻. The sample was diluted so as to fall within the range of the calibration curve. The resulting sample in an amount of 25 µL was injected, and subjected to IC measurement.

The results are tabulated in the Table below.

**[Table 1]**

| Mineral concentration [mg/kg] The values below each mineral are the quantitative lower limits. | | | | | | | | | | | | | | | | | |
|---|---|---|---|---|---|---|---|---|---|---|---|---|---|---|---|---|---|
| Example | Activated carbon | | Liquid extract | Temperature | Stir ring | | Concentrated | pH | Na | K | Ca | Mg | Zn | Fe | Si | Cl | SO₄²⁻ |
| | Type | [g] | [g] | [°C] | [rpm] | [min] | | | 0.01 | 0.1 | 0.001 | 0.001 | 0.001 | 0.001 | 0.01 | 0.05 | 0.03 |
| 1 | TAIKO CW | 30 | 400 | 90 | 100 | 15 | No | 9.46 | 62.59 | 390.7 | 0.454 | 0.175 | 0.000 | 0.066 | 15.59 | 88.87 | 14.97 |
| 2 | KURARAY COAL (registered trademark) GG | 30 | 400 | 90 | 100 | 15 | No | 9.81 | 69.60 | 474.5 | 0.699 | 0.347 | 0.000 | 0.080 | 17.43 | 0.70 | 1.74 |
| 3 | TAIKO CW | 30 | 130 | 90 | 100 | **10** | No | 9.15 | 133.30 | 1008.0 | 0.222 | 0.212 | 0.002 | 0.070 | 44.83 | 106.9 | 8.38 |
| 4 | TAIKO CW | 30 | 130 | 90 | 100 | **20** | No | 9.04 | 138.30 | 1012.0 | 0.189 | 0.181 | 0.002 | 0.079 | 51.42 | 266.6 | 9.01 |
| 5 | TAIKO CW | 30 | 130 | 90 | 100 | **40** | No | 9.09 | 139.20 | 997.0 | 0.293 | 0.201 | 0.001 | 0.106 | 57.74 | 278.4 | 9.34 |
| 6 | TAIKO CW | 30 | 130 | 90 | 100 | **80** | No | 9.29 | 131.80 | 948.0 | 0.223 | 0.314 | 0.003 | 0.133 | 65.90 | 292.2 | 9.23 |
| 7 | TAIKO CW | 30 | 400 | 90 | 100 | 5 | No | 10.61 | 43.18 | 292.0 | 0.524 | 0.678 | 0.015 | 0.247 | 12.62 | 87.1 | 3.59 |
| 8 | TAIKO CW | 30 | **200** | 90 | 100 | 5 | No | 10.43 | 95.90 | 671.4 | 0.976 | 0.520 | 0.015 | 0.213 | 22.69 | 174.2 | 5.35 |
| 9 | TAIKO CW | 30 | **130** | 90 | 100 | 5 | No | 10.32 | 115.6 | 870.0 | 0.908 | 0.675 | 0.021 | 0.343 | 39.42 | 294.1 | 9.34 |
| 10 | TAIKO CW | 30 | 400 | **90** | 100 | 5 | No | 10.52 | 47.12 | 322.0 | 0.499 | 0.606 | 0.009 | 0.335 | 14.82 | 93.9 | 3.54 |
| 11 | TAIKO CW | 30 | 400 | **60** | 100 | 5 | No | 10.56 | 51.30 | 342.2 | 0.528 | 0.232 | 0.023 | 0.111 | 8.02 | 88.2 | 3.28 |
| 12 | TAIKO CW | 30 | 400 | **30** | 100 | 5 | No | 10.12 | 44.92 | 304.0 | 0.559 | 0.165 | 0.008 | 0.054 | 3.57 | 83.2 | 2.96 |

Changing the activated carbon, the extraction time, the amount of the liquid extract with respect to the activated carbon, and the extraction temperature did not change the characteristics in that the potassium concentration was significantly high. In addition, with HCl used, a significant amount of chloride ions was extracted (data not shown), but the chloride ion concentration was low in any of Examples. In this regard, no heavy metal (lead, cadmium, arsenic, water silver, or the like) was detected in any of the above-mentioned Examples (data not shown).

### <Example 13: production of liquid concentrate>

Into a 1 L Erlenmeyer flask, 174 g of palm shell activated carbon ("TAIKO CW Type", not cleaned, manufactured by Futamura Chemical Co., Ltd.) and 753 g of distilled water heated to 30°C were introduced, and the resulting mixture was stirred with a stirring bar under heating at 30°C at 100 rpm for 5 minutes. The resulting suspension was filtrated with suction through a polyester mesh of 500 (25 µm), and the resulting filtrate was centrifuged at 3000 rpm for 10 minutes. After the centrifugation, the resulting supernatant was filtrated with suction through a paper filter to give a liquid mineral extract. The same operation was performed another two times. The resulting three liquid mineral extracts were mixed, and concentrated 62-fold using an evaporator to give the below-mentioned mineral concentrate extract.

The liquid mineral extract and the 62-fold-diluted mineral concentrate extract produced in Example 13 were analyzed in accordance with the above-mentioned method. The results are tabulated in the Table below.

**[Table 2]**

| Mineral concentration [mg/kg] The values below each mineral are the quantitative lower limits. | | | | | | | | | | | | | | | | | |
|---|---|---|---|---|---|---|---|---|---|---|---|---|---|---|---|---|---|
| Example | Activated carbon | | Liquid extract | Temperature | Stirring | | Concentrated | pH | Na | K | Ca | Mg | Zn | Fe | Si | Cl | SO₄²⁻ |
| | Type | [g] | [g] | [°C] | [rpm] | [min] | | | 0.01 | 0.1 | 0.001 | 0.001 | 0.001 | 0.001 | 0.01 | 0.05 | 0.03 |
| 13 | TAIKO CW | 521 | 2259 | 30 | 100 | 5 | No | 9.77 | 129.4 | 958.6 | 0.232 | 0.309 | 0.003 | 0.020 | 7.50 | 245.3 | 7.41 |
| | | | | | | | Yes | 9.76 | 121.0 | 941.6 | 0.237 | 0.323 | 0.005 | 0.020 | 7.05 | 242.2 | 6.92 |

Undergoing the concentrating conditions did not change the characteristics in that the potassium concentration was high, and that the sodium concentration and the chloride ion concentration were low.

### <Example 14: production of mineral concentrate extract from palm shell activated carbon>

Into a 1 L Erlenmeyer flask, 200 g of palm shell activated carbon ("TAIKO CW Type", not cleaned, manufactured by Futamura Chemical Co., Ltd.) and 1500 g of distilled water heated to 90°C were introduced, and the resulting mixture was stirred with a stirring bar under heating at 90°C at 100 rpm for 15 minutes. The resulting suspension was filtrated with suction through a polyester mesh of 500 (25 µm), and the resulting filtrate was centrifuged at 3000 rpm for 10 minutes. After the centrifugation, the resulting supernatant was filtrated with suction through a paper filter to give a liquid mineral extract. The resulting liquid mineral extract was concentrated 14-fold using an evaporator to give the below-mentioned mineral concentrate extract.

**[Table 3]**

| - Concentration of ions of mineral concentrate extract | | | |
|---|---|---|---|
| | Ion component | Concentration (mg/L) | |
| | Na | 1,650 | |
| | K | 11,451 | |
| | Mg | 1 | |
| | Ca | 2 | |
| | Fe | 2 | |
| | Zn | 3 | |
| | Cl⁻ | 2,442 | |
| | SO₄²⁻ | 230 | |

### <Example 15: buffer capacity evaluation - I>

### (1) Production of evaluation sample

The mineral concentrate extract given as above-mentioned was added to ultrapure water (MilliQ water) in such a manner that the resulting water had the respective potassium concentrations as below-mentioned, whereby evaluation samples were produced.

**[Table 4]**

| | | | | | | | | |
|---|---|---|---|---|---|---|---|---|
| Amount of liquid extract | ml | 100 | 100 | 100 | 100 | 100 | 100 | 100 |
| Amount of extract added | ml | 0.076 | 0.152 | 0.303 | 0.607 | 0.758 | 1.516 | 4.549 |
| Total amount of liquid | ml | 100.000 | 100.000 | 100.000 | 100.000 | 100.000 | 100.000 | 100.000 |
| K concentration | mg/L | 10 | 20 | 40 | 80 | 100 | 200 | 600 |

### (2) pH measurement

Besides the liquid extracts given as above-mentioned, the following samples were made ready for use in Comparative Example. To 100 ml of each sample, 0.1 N HCl was added 1 ml by 1 ml with stirring with a stirring bar, and the pH was measured.
- KOH
- Commercially available alkaline ionized water (Na: 8.0 mg/l, K: 1.6 mg/l, Ca: 13 mg/l, Mg: 6.4 mg/l, pH value: 8.8 to 9.4)

The buffer capacity was defined as a ratio (B)/(A), assuming that the amount of 0.1 M hydrochloric acid solution with which 100 g of a sodium hydroxide solution adjusted to a pH of 9.2 was titrated from a pH of 9.2 to a pH of 3.0 was (A) mL, and that the amount of 0.1 M hydrochloric acid solution with which the mineral-containing aqueous composition was titrated from a pH of 9.2 to a pH of 3.0 was (B) mL.

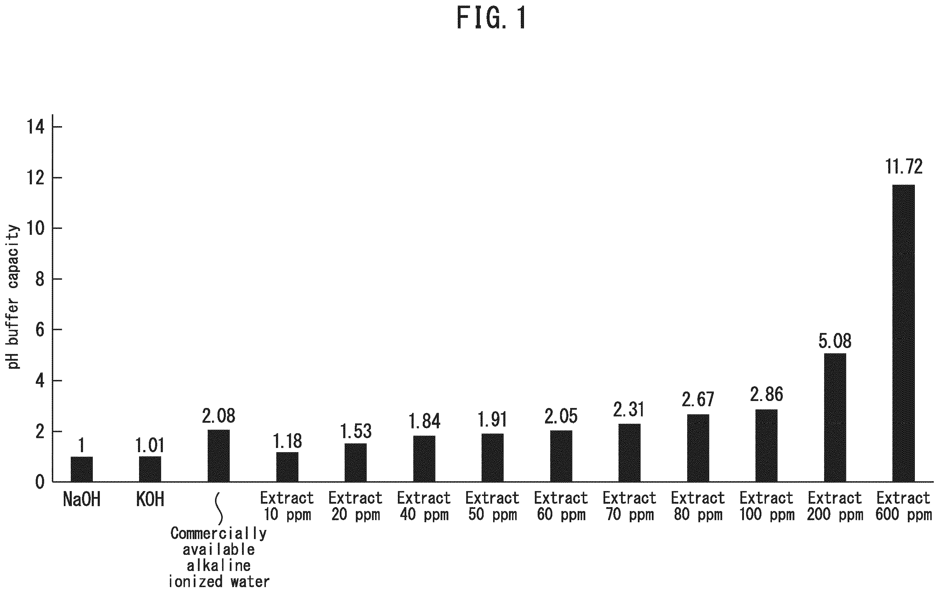

As illustrated in FIG. 1, the water containing the added mineral concentrate extract derived from palm shell activated carbon proved to have an excellent buffer capacity.

### <Example 16: buffer capacity evaluation - II>

### (1) Comparative Example and production of evaluation sample

As Comparative Examples, purified water (tap water treated with a water purifier manufactured by Waterstand Co., Ltd.) and the same commercially available alkaline ionized water as in Example 15 were made ready for use. In addition, the mineral concentrate extract given in Example 14 was added to purified water (the same as above-mentioned) in such a manner that the resulting water had a potassium concentration of 100 ppm, whereby an evaluation sample was produced.

### (2) pH measurement

The buffer capacity of each of the samples given as above-mentioned was evaluated in the same manner as in Example 15. In other words, 0.1 N HCl was added 1 ml by 1 ml to 100 ml of each sample with stirring with a stirring bar, and the pH was measured.

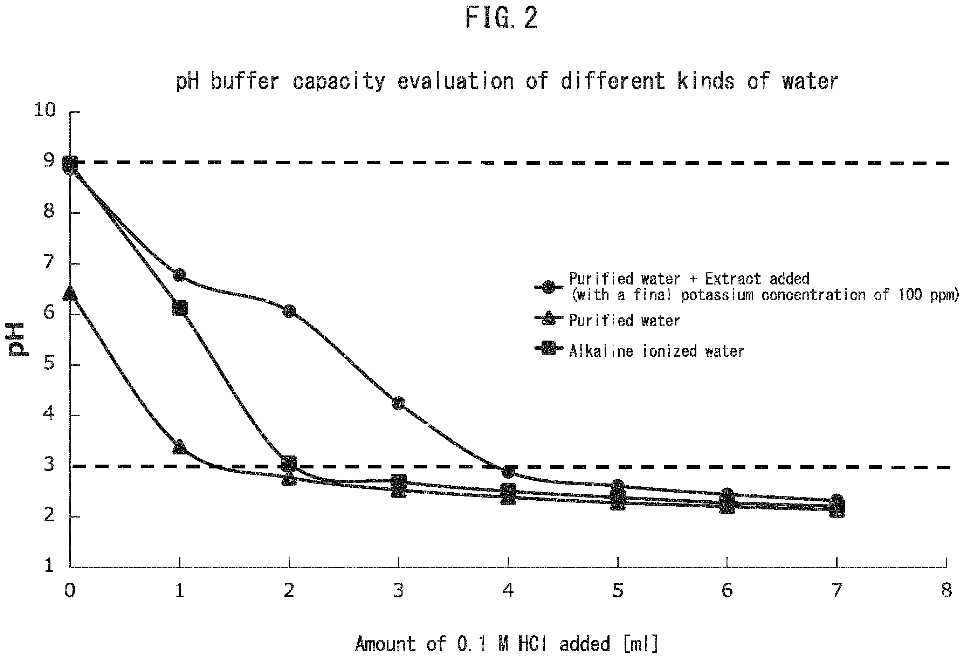

As illustrated in FIG. 2, the water that was purified tap water containing the added mineral concentrate extract derived from palm shell activated carbon proved to have an excellent buffer capacity, compared with the purified water and the alkaline ionized water.

### <Example 17: production of mineral concentrate extract from palm shell activated carbon>

### = Pilot scale =

Pure water in an amount of 180 L was allowed to pass through 40 kg of palm shell activated carbon ("TAIKO", not cleaned with hydrochloric acid, manufactured by Futamura Chemical Co., Ltd.), and the resulting suspension was clarified with a mesh and by centrifugation to give a liquid mineral extract. The liquid mineral extract was concentrated 92-fold under reduced pressure using a centrifugal thin-film vacuum evaporator, and the resulting liquid concentrate was clarified by centrifugation and through a paper filter. With this resulting liquid, a 1 L plastic pouch was packed, and the liquid was heat-treated at 85°C for 30 minutes to give a mineral concentrate extract. With the resulting mineral concentrate extract, the potassium ion concentration, sodium ion concentration, calcium ion concentration, and magnesium ion concentration were analyzed by ICP atomic emission spectroscopy, the chloride ion concentration was analyzed by ion chromatography, and the TOC was analyzed by total organic carbon measurement. In addition, the resulting mineral concentrate extract was stored under refrigeration for two weeks, and then, the degree of turbidity was evaluated by visual observation in accordance with the following five-step rating: "-" (exhibiting high transparency and having no recognizable suspended matter or precipitate); "+" (having a slight amount of recognizable suspended matter and/or precipitate); "++" (having a large amount of recognizable suspended matter and/or aggregate); "+++" (having an even larger amount of recognizable suspended matter and/or aggregate and exhibiting lost transparency); "++++" (having a large amount of suspended matter and deposited aggregate and exhibiting low transparency).

### <Example 18: production of mineral concentrate extract from palm shell activated carbon>

### = Laboratory small scale =

To 200 g of palm shell activated carbon (Granular SHIRASAGI, not cleaned with hydrochloric acid, manufactured by Osaka Gas Chemicals Co., Ltd.), 910 g of distilled water was added, and the resulting mixture was stirred with a stirring bar under heating at 30°C at 100 rpm for 20 minutes. The resulting suspension was filtrated with suction through a paper filter (an ADVANTEC quantitative paper filter No. 5C, 55 mm in diameter, manufactured by Toyo Roshi Kaisha, Ltd.), and the resulting filtrate was further filtrated with suction through a paper filter (MERCK Omnipore PTFE Membrane, 5.0 µm, 47 mm in diameter) to give a liquid mineral extract. This operation was repeated a plurality of times until a sufficient amount of the liquid mineral extract was given, and the whole liquid mineral extract was mixed, and then concentrated 50-fold under reduced pressure using a rotary evaporator. The resulting liquid concentrate was filtrated through a paper filter (an ADVANTEC 25ASO20AN, 0.2 µm, manufactured by Toyo Roshi Kaisha, Ltd.) to give a mineral concentrate extract. Hydrochloric acid was added to this liquid mineral concentrate, the pH of which was thus adjusted to approximately 9.5, and 10 mL of the resulting mixture was dispensed into a vial, and stored under refrigeration for 2 days. Then, the mixture was filtrated under cooling through a paper filter (an ADVANTEC 25ASO20AN, 0.2 µm, manufactured by Toyo Roshi Kaisha, Ltd.), and the resulting filtrate was heat-treated at 80°C for 30 minutes to give a mineral concentrate extract. With the resulting mineral concentrate extract, the potassium ion concentration, sodium ion concentration, calcium ion concentration, and magnesium ion concentration were analyzed by inductively coupled plasma-atomic emission spectroscopy (ICP-AES), and the chloride ion concentration and the sulfate ion concentration were analyzed by ion chromatography (IC). In addition, the resulting mineral concentrate extract was stored under refrigeration for two weeks, and then, the degree of turbidity was evaluated by visual observation in accordance with the following five-step rating: "-" (exhibiting high transparency and having no recognizable suspended matter or precipitate); "+" (having a slight amount of recognizable suspended matter and/or precipitate); "++" (having a large amount of recognizable suspended matter and/or aggregate); "+++" (having an even larger amount of recognizable suspended matter and/or aggregate and exhibiting lost transparency); "++++" (having a large amount of suspended matter and deposited aggregate and exhibiting low transparency).

### <Example 19: production of mineral concentrate extract from palm shell activated carbon>

### = Laboratory large scale =

To 800 g of palm shell activated carbon (Granular SHIRASAGI, not cleaned with hydrochloric acid, manufactured by Osaka Gas Chemicals Co., Ltd.), 3660 g of distilled water was added, and the resulting mixture was stirred under heating at 30°C for 15 minutes. The resulting suspension was filtrated with suction through a paper filter (an ADVANTEC A080A090C, manufactured by Toyo Roshi Kaisha, Ltd.) to give a liquid mineral extract. This operation was repeated a plurality of times until a sufficient amount of the liquid mineral extract was given, and the whole liquid mineral extract was mixed, and then concentrated 60-fold under reduced pressure using a rotary evaporator. The resulting liquid concentrate was filtrated through a paper filter (an ADVANTEC A080A090C, manufactured by Toyo Roshi Kaisha, Ltd.) to give a mineral concentrate extract. The resulting mixture in an amount of 10 mL was dispensed into a vial, and stored under refrigeration for 2 days. Then, the mixture was filtrated under cooling through a paper filter (an ADVANTEC A080A090C, manufactured by Toyo Roshi Kaisha, Ltd.). Hydrochloric acid was added to the resulting filtrate, the pH of which was thus adjusted to approximately 9.5. The resulting mixture was diluted with pure water so as to have a potassium ion concentration of approximately 100000 ppm. This resulting mixture was heat-treated at 80°C for 30 minutes to give a mineral concentrate extract. With the resulting mineral concentrate extract, the potassium ion concentration, sodium ion concentration, calcium ion concentration, magnesium ion concentration, and sulfate ion concentration were analyzed by ion chromatography (IC), the chloride ion concentration was analyzed by ion chromatography, and the TOC was analyzed by total organic carbon measurement. In addition, the resulting mineral concentrate extract was stored under refrigeration for two weeks, and then, the degree of turbidity was evaluated by visual observation in accordance with the following five-step rating: "-" (exhibiting high transparency and having no recognizable suspended matter or precipitate); "+" (having a slight amount of recognizable suspended matter and/or precipitate); "++" (having a large amount of recognizable suspended matter and/or aggregate); "+++" (having an even larger amount of recognizable suspended matter and/or aggregate and exhibiting lost transparency); "++++" (having a large amount of suspended matter and deposited aggregate and exhibiting low transparency).

### <Example 20: production of mineral concentrate extract from palm shell activated carbon>

### = Pilot scale =

Into a 2500-L conical tank, 360 kg of palm shell activated carbon ("Granular SHIRASAGI", not cleaned, manufactured by Osaka Gas Chemicals Co., Ltd.) and 1620 kg of 35°C pure water were introduced, and the resulting mixture was stirred for 15 minutes. The resulting suspension was clarified with a shaking sieve, by centrifugation, and by filtration through a paper filter to give a liquid mineral extract. The liquid mineral extract was concentrated 60-fold under reduced pressure using a centrifugal thin-film vacuum evaporator, and the resulting liquid concentrate was filtrated through a paper filter to give a mineral concentrate extract. A drum was packed with the extract, stored under refrigeration for 2 days, and then filtrated under cooling through a paper filter. Hydrochloric acid was added to the resulting filtrate, the pH of which was thus adjusted to approximately 9.5. The resulting mixture was diluted with pure water so as to have a potassium ion concentration of approximately 100000 ppm. This resulting mixture was heat-treated at 130°C for 30 seconds to give a mineral concentrate extract. With the resulting mineral concentrate extract, the potassium ion concentration, sodium ion concentration, calcium ion concentration, magnesium ion concentration, and sulfate ion concentration were analyzed by ion chromatography (IC), the chloride ion concentration was analyzed by ion chromatography, and the TOC was analyzed by combustion oxidation-infrared TOC analysis. In addition, the resulting mineral concentrate extract was stored under refrigeration for two weeks, and then, the degree of turbidity was evaluated by visual observation in accordance with the following five-step rating: "-" (exhibiting high transparency and having no recognizable suspended matter or precipitate); "+" (having a slight amount of recognizable suspended matter and/or precipitate); "++" (having a large amount of recognizable suspended matter and/or aggregate); "+++" (having an even larger amount of recognizable suspended matter and/or aggregate and exhibiting lost transparency); "++++" (having a large amount of suspended matter and deposited aggregate and exhibiting low transparency). Furthermore, the NTU turbidity was measured using a turbidimeter (2100AN TURBISIMETRER, manufactured by Hach Company).

The results of Examples 17 to 20 are tabulated in Table 5. In terms of the components of the mineral extract, a mineral extract having a potassium concentration of 60994 ppm, a chloride ion concentration of 3030 ppm, and a pH of 11.1 was given in Example 17, a mineral extract having a potassium concentration of 87500 ppm, a chloride ion concentration of 32890 ppm, and a pH of 9.50 was given in Example 18, a mineral extract having a potassium concentration of 100000 ppm, a chloride ion concentration of 13132 ppm, and a pH of 9.51 was given in Example 19, and a mineral extract having a potassium concentration of 111747 ppm, a chloride ion concentration of 8545 ppm, and a pH of 9.48 was given in Example 20. In addition, in terms of turbidity, Example 17 was rated "++++" (having a large amount of suspended matter and deposited aggregate and exhibiting low transparency), and on the other hand, all of Example 18, Example 19, and Example 20, which underwent storage under refrigeration and filtration under cooling, were rated "++" (having a large amount of recognizable suspended matter and/or aggregate). In particular, Example 18, in which a pH adjustment was made before storage under refrigeration and filtration under cooling, was rated "-" (exhibiting high transparency and having no recognizable suspended matter or precipitate). This has proved that the storage under refrigeration and the filtration under cooling are desirable in order to give a mineral extract having high transparency, and a pH adjustment, if made, is desirably made before the storage under refrigeration and the filtration under cooling.

**[Table 5]**

| | pH before adjustment | pH after adjustment | Na (ppm) | K (ppm) | Ca (ppm) | Mg (ppm) | Cl (ppm) | SO₄ (ppm) | TOC (ppm) | Turbidity | Turbidity (visual observation) |
|---|---|---|---|---|---|---|---|---|---|---|---|
| Example 17 | 11.1 | | 5,627 | 60,994 | 20 | 5 | 3,030 | not measured | 186 | not measured | +++ |
| Example 18 | 9.95 | 9.5 | 7,100 | 87,500 | 830 | 44 | 32,890 | 1,481 | not measured | not measured | - |
| Example 19 | 9.86 | 9.51 | 9,000 | 100,000 | 190 | 185 | 13,132 | 90 | 210 | not measured | + |
| Example 20 | 9.58 | 9.48 | 9.531 | 111,747 | 99 | 66 | 8,545 | 0 | 140 | 47.1 | ++ |

### <Example 21: organoleptic evaluation>

The mineral concentrate extract produced in Example 1 or potassium carbonate was added to purified water in such a manner that the final potassium concentration was 50 to 300 ppm, to give a sample of potable mineral water, as listed in the Table below. In addition, purified water as a control was made ready for use. The purified water used was tap water treated using a commercially available general-purpose water purifier (from the water, chlorine smell and the like were removed with activated carbon).

**[Table 6]**

| | Na (ppm) | K (ppm) | Ca (ppm) | Mg (ppm) | Cl⁻ (ppm) | SO₄²⁻ (ppm) |
|---|---|---|---|---|---|---|
| A) Purified water | 6.9 | 1.3 | 16.0 | 4.5 | 6.8 | 21.0 |
| B) Purified water + 0.43 v/v% extract | 13.9 | 50 | 15.9 | 4.5 | 17.2 | 21.9 |
| C) Purified water + 0.86 v/v% extract | 21.1 | 100 | 15.9 | 4.5 | 27.8 | 22.8 |
| D) Purified water + 1.74 v/v% extract | 35.4 | 200 | 15.8 | 4.4 | 49.1 | 24.6 |
| E) Purified water + 2.61 v/v% extract | 49.8 | 300 | 15.6 | 4.4 | 70.4 | 26.5 |
| F) Purified water + 86.1 ppm potassium carbonate | 6.9 | 50 | 16.0 | 4.5 | 6.8 | 21.0 |
| G) Purified water + 174.5 ppm potassium carbonate | 6.9 | 100 | 16.0 | 4.5 | 6.8 | 21.0 |
| H) Purified water + 351.3 ppm potassium carbonate | 6.9 | 200 | 16.0 | 4.5 | 6.8 | 21.0 |
| I) Purified water + 528.1 ppm potassium carbonate | 6.9 | 300 | 16.0 | 4.5 | 6.8 | 21.0 |

The samples as above-mentioned underwent an organoleptic evaluation by four trained evaluation panelists. For the organoleptic evaluation, the evaluation criteria were preliminarily compared and adjusted among the evaluation panelists, and the "mild taste" and "odd taste" of the water were evaluated in comparison with the control. Then, the evaluation scores of the panelists were averaged.

The "mild taste" was defined as a taste that gives a good mouth-feel, no stimulus, and a round flavor, and based on the following four-step evaluation scoring (0 point = equal to the control, 1 point = a little mild, 2 points = mild, and 3 points = very mild). This means that the positively larger the value, the more strengthened the mild taste.

The "odd taste" was defined as an unpleasant flavor such as bitterness or acridity, and based on the following four-step evaluation scoring (0 point = equal to the control, -1 point = a little odd, -2 points = odd, and -3 points = very odd). This means that the negatively larger the value, the stronger the odd taste.

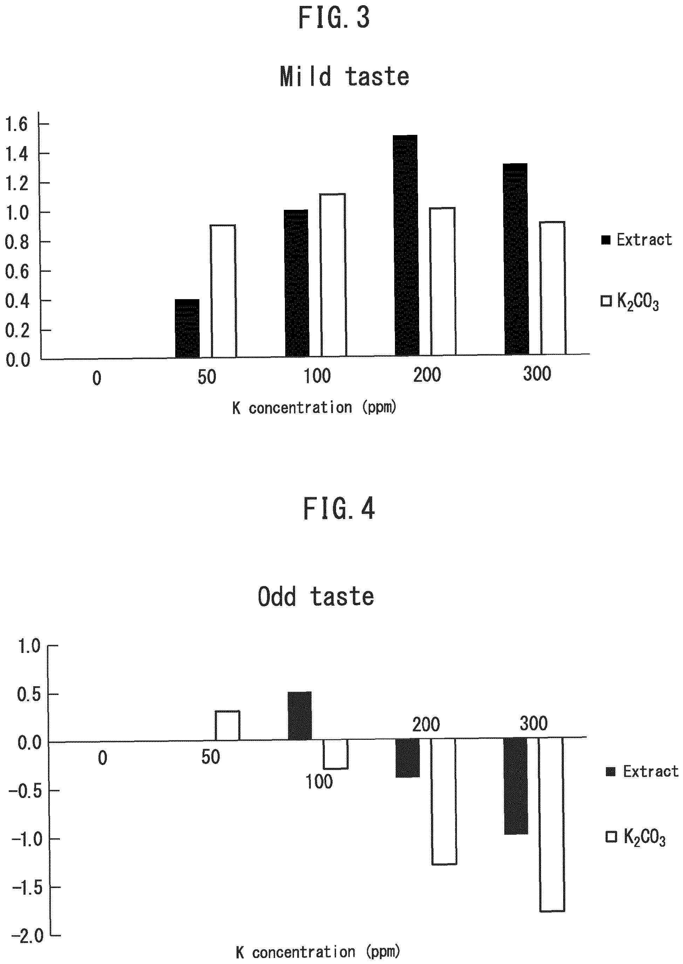

As understood from the organoleptic evaluation regarding the "mild taste" (FIG. 3), adding the mineral concentrate extract derived from palm shell activated carbon makes it possible to give a milder taste than purified water. In addition, in the case of comparison between the sample C and the sample G in the Table above, all the evaluation panelists answered that the water containing the added mineral concentrate extract was milder than the aqueous potassium carbonate solution, and in the case of comparison between the sample D and the sample H in the Table above, half or more of the evaluation panelists answered that the water containing the added mineral concentrate extract was milder than the aqueous potassium carbonate solution.

As understood from the organoleptic evaluation regarding the "odd taste" (FIG. 4), the water containing the added mineral concentrate extract derived from palm shell activated carbon is less odd than the potassium carbonate solution in cases where both of them have the same potassium concentration. In addition, in the case of comparison between the sample C and the sample G in the Table above, half or more of the evaluation panelists answered that the water containing the added mineral concentrate extract was less odd than the aqueous potassium carbonate solution, and in the case of comparison between the sample D and the sample H in the Table above, all the evaluation panelists answered that the water containing the added mineral concentrate extract was less odd than the aqueous potassium carbonate solution.

### <Example 22: organoleptic evaluation of water - influence of potassium concentration>

As water, purified water (tap water treated using a water purifier) and tap water were made ready for use. An organoleptic evaluation was performed using each kind of water to which a mineral concentrate extract (having a potassium concentration of 104000 ppm) given in the same manner as in Example 17 was added in such a manner that the concentration of potassium added to the water was as below-mentioned.

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 7]**

| K concentration (mg/L = ppm) | 50 | 60 | 70 | 80 | 90 | 100 |
|---|---|---|---|---|---|---|
| Tap water | ○ | ⊚ | ⊚ | ⊚ | ⊚ | ⊚ |
| Purified water | ○ | ⊚ | ⊚ | ⊚ | ⊚ | ⊚ |

The purified water and the tap water that each contained the added mineral concentrate extract had a significantly improved flavor in the potassium concentration range of from 50 to 100 ppm. In particular, the tap water verified a significant decrease in the chlorine smell in the potassium concentration range of from 50 to 100 ppm, compared with the water yet to contain the added mineral concentrate extract.

### <Example 23: organoleptic evaluation of water - influence of pH>

As water, purified water (tap water treated using a water purifier) and tap water were made ready for use. A mineral concentrate extract (having a potassium concentration of 53375 ppm) given in the same manner as in Example 17 was supplemented with hydrochloric acid to have a pH adjusted (to 11.2, 10.2, 9.2 and 8.1), and then added to water in such a manner that the concentration of potassium added to the water was as below-mentioned. Then, the resulting water underwent an organoleptic evaluation.

The organoleptic evaluation was performed by five trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 8]**

| K concentration (mg/L = ppm) | | 50 | 100 | 200 | 300 | 450 |
|---|---|---|---|---|---|---|
| Tap water | pH 11.2 | ○ | △ | × | × | × |
| | pH 10.2 | ○ | ○ | ○ | △ | × |
| | pH 9.2 | ⊚ | ⊚ | ⊚ | ○ | △ |
| | pH 8.1 | ○ | ○ | ○ | ○ | △ |
| Purified water | pH 11.2 | ○ | ○ | △ | × | × |
| | pH 10.2 | ○ | ○ | ○ | △ | △ |
| | pH 9.2 | ⊚ | ⊚ | ○ | ○ | △ |
| | pH 8.1 | ○ | ○ | ○ | △ | △ |

With the mineral water containing the added mineral concentrate extract and having a pH adjusted to 8.1 to 11.2, particularly 8.1 to 10.2, the fragrance and flavor were significantly improved in a wide potassium concentration range. In addition, the tap water verified a significant decrease in the chlorine smell at any of the pH values in the potassium concentration range of 50 ppm or more, compared with the water yet to contain the added mineral concentrate extract. From the pH values and the potassium concentrations, a pH-potassium concentration range for good fragrance and flavor was obtained. Also with the purified water, a pH-potassium concentration range for good fragrance and flavor was obtained from the pH values and the potassium concentrations.

### <Example 24: improvement effect on taste of drink in ice>

As water, purified water (tap water treated using a water purifier), tap water, and commercially available mineral water (natural water) were made ready for use. To each kind of water, a mineral concentrate extract (having a potassium concentration of 53375 ppm) given in the same manner as in Example 17 was added in such a manner that the concentration of potassium added to the water was as below-mentioned. The resulting water in an amount of 10 ml was placed in a cup, and frozen overnight. Five minutes after the resulting ice was taken out, an organoleptic evaluation of the flavor of the ice was performed.

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 9]**

| K concentration (mg/L = ppm) | 50 | 100 | 300 | 500 |
|---|---|---|---|---|
| Tap water | ○ | ⊚ | △ | △ |
| Purified water | ○ | ⊚ | △ | △ |
| Natural water | ○ | ⊚ | △ | × |

The ice itself produced with each of the purified water, the tap water, and the commercially available mineral water (natural water) that each contained the added mineral concentrate extract had a significantly improved flavor in the potassium concentration range of from 50 to 100 ppm.

The ice given as above-mentioned was added to 360 µl of whiskey having an alcohol concentration of 40%, and the resulting whiskey underwent an organoleptic evaluation of the flavor (tastiness and fragrance).

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 10]**

| K concentration (mg/L = ppm) | | 50 | 100 | 300 | 500 |
|---|---|---|---|---|---|
| Tastiness | Whiskey + tap water ice | ○ | ○ | × | × |
| | Whiskey + purified water ice | ○ | ○ | × | × |
| | Whiskey + natural water ice | ○ | ○ | △ | △ |
| Fragrance | Whiskey + tap water ice | ○ | ○ | △ | △ |
| | Whiskey + purified water ice | ○ | ○ | △ | △ |
| | Whiskey + natural water ice | ○ | ⊚ | △ | △ |

The whiskey with the added ice produced with each of the purified water, the tap water, and the commercially available mineral water (natural water) that each contained the added mineral concentrate extract had a significantly improved whiskey flavor in the potassium concentration range of from 50 to 100 ppm, compared with the ice containing no added mineral concentrate extract.

The ice given as above-mentioned was added to 1400 µl of Japanese distilled spirit having an alcohol concentration of 25%, and the resulting Japanese distilled spirit underwent an organoleptic evaluation of the flavor (tastiness and fragrance).

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚

### for 3.1 or more.

**[Table 11]**

| K concentration (mg/L = ppm) | | 50 | 100 | 300 | 500 |
|---|---|---|---|---|---|
| Tastiness | Japanese distilled spirit + tap water ice | ○ | ○ | △ | △ |
| | Japanese distilled spirit + purified water ice | ○ | ○ | △ | × |
| | Japanese distilled spirit + natural water ice | ○ | ○ | △ | × |
| Fragrance | Japanese distilled spirit + tap water ice | ○ | ○ | △ | △ |
| | Japanese distilled spirit + purified water ice | ○ | ○ | △ | × |
| | Japanese distilled spirit + natural water ice | ○ | ○ | △ | × |

The Japanese distilled spirit with the added ice produced with each of the purified water, the tap water, and the commercially available mineral water (natural water) that each contained the added mineral concentrate extract had a significantly improved Japanese distilled spirit flavor in the potassium concentration range of from 50 to 100 ppm, compared with the ice containing no added mineral concentrate extract.

The ice given as above-mentioned was added to 1400 µl of lemon sour, and the resulting lemon sour underwent an organoleptic evaluation of the flavor (tastiness and fragrance).

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 12]**

| K concentration (mg/L = ppm) | | 50 | 100 | 300 | 500 |
|---|---|---|---|---|---|
| Tastiness | Lemon sour + tap water ice | ○ | ○ | ○ | △ |
| | Lemon sour + purified water ice | ○ | ○ | ○ | △ |
| | Lemon sour + natural water ice | ○ | ○ | ○ | △ |
| Fragrance | Lemon sour + tap water ice | ○ | ○ | ○ | ○ |
| | Lemon sour + purified water ice | ○ | ○ | ○ | ○ |
| | Lemon sour + natural water ice | ○ | ○ | ○ | ○ |

The lemon sour with the added ice produced with each of the purified water, the tap water, and the commercially available mineral water (natural water) that each contained the added mineral concentrate extract had a significantly improved lemon sour flavor in the potassium concentration range of from 50 to 500 ppm, compared with the ice containing no added mineral concentrate extract.

The ice produced with the tap water verified a significant decrease in the chlorine smell in the potassium concentration range of from 50 to 100 ppm, compared with the water containing no added mineral concentrate extract.

### <Example 25: organoleptic evaluation of extract drink>

As water, purified water (tap water treated using a water purifier), tap water, and commercially available mineral water (natural water) were made ready for use. To each kind of water, a mineral concentrate extract (having a potassium concentration of 53375 ppm) given in the same manner as in Example 17 was added in such a manner that the concentration of potassium added to the water was as below-mentioned. The resulting water was boiled, and used as extraction water (100 ml) for coffee and green tea.

Coffee beans made in Brazil were weighed in an amount of 10 g out into each cup and milled. On the coffee beans milled, the above-mentioned boiled extract water was poured to extract coffee. The resulting coffee was left to stand for 4 minutes, and underwent an organoleptic evaluation of the liquid coffee extract.

An organoleptic evaluation was performed using the following four kinds of coffee; without milk and sugar; with milk (milk added at 500 µl per 15 ml); with sugar (granulated sugar added at 50 ml per 3 g); and with milk and sugar (granulated sugar added at 166 µl and milk added at 50 ml, per 3 g). Four trained evaluation panelists preliminarily compared and adjusted the evaluation criteria among the evaluation panelists before the evaluation. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 13]**

| K concentration (mg/L = ppm) | | 50 | 100 | 300 | 500 |
|---|---|---|---|---|---|
| Without milk and sugar | tap water | ○ | △ | △ | △ |
| | Purified water | ○ | △ | △ | △ |
| | Natural water | ○ | ○ | △ | △ |
| With milk | Tap water | ○ | ○ | △ | × |
| | purified water | ○ | ○ | △ | △ |
| | Natural water | ○ | △ | △ | × |
| With sugar | Tap water | ○ | ○ | ○ | △ |
| | Purified water | ○ | ○ | ○ | ○ |
| | Natural water | ○ | ○ | ○ | △ |
| With milk and sugar | Tap water | ○ | ○ | △ | △ |
| | Purified water | ○ | ○ | ○ | △ |
| | Natural water | ○ | ○ | ○ | △ |

The coffee extracted using each of purified water, tap water, and commercially available mineral water (natural water) that each contained the added mineral concentrate extract and was used as an extraction solvent had a significantly improved coffee flavor in the potassium concentration range of from 50 to 300 ppm, compared with the coffee extracted using the extraction solvent containing no added mineral concentrate extract.

Green tea leaves were weighed in an amount of 2 g out into each cup. On the tea leaves, the above-mentioned boiled extract water was poured to extract green tea. The resulting green tea was left to stand for 3 minutes, and underwent an organoleptic evaluation of the liquid green tea extract.

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 14]**

| K concentration (mg/L = ppm) | 50 | 100 | 300 | 500 |
|---|---|---|---|---|
| Tap water | ⊚ | ○ | △ | × |
| Purified water | ○ | △ | △ | × |
| Natural water | ○ | △ | △ | × |

The green tea extracted using each of purified water, tap water, and commercially available mineral water (natural water) that each contained the added mineral concentrate extract and was used as an extraction solvent had a significantly improved tea flavor in the potassium concentration range of from 50 to 100 ppm, compared with the green tea extracted using the extraction solvent containing no added mineral concentrate extract.

### <Example 26: organoleptic evaluation of different kinds of drinks>

An organoleptic evaluation was performed using different kinds of drinks to which a mineral concentrate extract (having a potassium concentration of 96900 ppm) given in the same manner as in Example 17 was added in such a manner that the concentration of potassium added to the drink was as below-mentioned.

The organoleptic evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but having very poor fragrance and flavor; 1 point = changed but having poor fragrance and flavor; 2 points = not changed; 3 points = changed and having good fragrance and flavor; and 4 points = changed and having very good fragrance and flavor) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 15-1]**

| K concentration (mg/L = ppm) | 50 | 100 | 300 | 450 | 600 |
|---|---|---|---|---|---|
| Beer (ALC. 5.5%) | ○ | ○ | ○ | △ | △ |
| Citrus alcoholic drink (ALC. 9%) | ○ | ○ | ○ | △ | △ |
| Nonalcoholic beer (ALC. 0%) | ○ | ○ | ○ | △ | △ |
| Whiskey (ALC. 7%) | ○ | ○ | ○ | △ | △ |
| Milky alcoholic drink (ALC. 3%) | ⊚ | ○ | ○ | ○ | ○ |
| Fruit (peach) alcoholic drink (ALC. 3%) | ○ | ○ | ○ | △ | △ |
| Lemon alcoholic drink (ALC. 9%) | ○ | ⊚ | ○ | ○ | △ |
| Lemon alcoholic drink (ALC. 7%) | ⊚ | ○ | ○ | ○ | △ |

The Table above verifies that the alcoholic drink verified containing the added mineral concentrate extract had a significantly improved flavor in the potassium concentration range of from 50 to 600 ppm, particularly from 50 to 100 ppm. In addition, the nonalcoholic beer had a significantly improved flavor in the potassium concentration range of from 50 to 300 ppm.

**[Table 15-2]**

| K concentration (mg/L = ppm) | 50 | 100 | 300 | 450 |
|---|---|---|---|---|
| Cola drink | ○ | ○ | △ | △ |
| Lemon carbonated drink | ○ | ○ | △ | △ |
| Orange juice drink | ○ | ○ | ○ | △ |
| Green tea drink | ○ | ○ | △ | △ |
| Barley tea drink | ○ | △ | △ | △ |
| Black coffee drink | ○ | ○ | ○ | △ |
| Black tee drink with milk | ○ | ○ | ○ | △ |

The mineral concentrate extract was added to different kinds of drinks. The cola drink or lemon carbonated drink had a significantly improved flavor in the potassium concentration range of from 50 to 100 ppm, the orange juice drink had a significantly improved flavor in the potassium concentration range of from 50 to 300 ppm, the green tea drink or barley tea drink had a significantly improved flavor in the potassium concentration range of from 50 to 100 ppm, the black coffee drink had a significantly improved flavor in the potassium concentration range of from 50 to 300 ppm, and the black tea drink with milk had a significantly improved flavor in the potassium concentration range of from 50 to 300 ppm.

### <Example 27: evaluation of foam quality of carbonated drink>

As water, purified water (tap water treated using a water purifier) and tap water were made ready for use. To each kind of water, a mineral concentrate extract (having a potassium concentration of 104000 ppm) given in the same manner as in Example 17 was added and adjusted in such a manner that the concentration of potassium added to the water was as below-mentioned. Then, the resulting water was carbonated using a soda siphon set to an equal gas pressure of 2.1±0.2 kg/cm², and the resulting drink carbonated was used as a sample. The foam quality (the "fineness of the foam", the "swallowability of the drink carbonated", and the "crispness of the aftertaste") of each sample was evaluated.

The evaluation was performed by four trained evaluation panelists, who preliminarily compared and adjusted the evaluation criteria among the evaluation panelists. In the evaluation, water containing no added mineral concentrate extract was used as a control. The scores given by the panelists on the basis of the following four-step evaluation scoring (0 point = changed but very poor; 1 point = changed but poor; 2 points = not changed; 3 points = changed and good; and 4 points = changed and very good) were totaled, and the average of the points was calculated. The rating was × for the average value of 1 or less, the rating was Δ for 1.1 or more and 2 or less, the rating was o for 2.1 or more and 3 or less, and the rating was ⊚ for 3.1 or more.

**[Table 16]**

| K concentration (mg/L = ppm) | | 50 | 100 | 300 |
|---|---|---|---|---|
| Tap water | Fineness of foam | ⊚ | ⊚ | ⊚ |
| | Swallowability of drink carbonated | ⊚ | ⊚ | ⊚ |
| | Crispness of aftertaste | ⊚ | ⊚ | ⊚ |
| Purified water | Fineness of foam | ⊚ | ⊚ | ⊚ |
| | Swallowability of drink carbonated | ○ | ⊚ | ⊚ |
| | Crispness of aftertaste | ⊚ | ⊚ | ⊚ |

The purified water and the tap water that each contained the added mineral concentrate extract and was carbonated had a significantly improved foam quality in the potassium concentration range of from 50 to 300 ppm.

## Claims

1. A liquid mineral concentrate composition comprising potassium ions the concentration of which is the highest of the metal ions present in the liquid mineral concentrate composition.

2. The liquid mineral concentrate composition according to claim 1, wherein the amount of chloride ions contained in the liquid mineral concentrate composition is 50% or less of the potassium ion concentration.

3. The liquid mineral concentrate composition according to claim 1 or 2, wherein the amount of calcium ions contained in the liquid mineral concentrate composition is 2.0% or less of the potassium ion concentration.

4. The liquid mineral concentrate composition according to any one of claims 1 to 3,
wherein the amount of magnesium ions contained in the liquid mineral concentrate composition is 1.0% or less of the potassium ion concentration.

5. The liquid mineral concentrate composition according to any one of claims 1 to 4,
wherein the amount of sodium contained in the liquid mineral concentrate composition is 5 to 45% of the potassium ion concentration.

6. The liquid mineral concentrate composition according to any one of claims 1 to 5, comprising a liquid extract from activated carbon of a plant-derived raw material.

7. The liquid mineral concentrate composition according to claim 6, wherein the plant-derived raw material is selected from the following: fruit shells of coconut palms, palms, almonds, walnuts, or plums; woods selected from sawdust, charcoal, resins, and lignin; sawdust ash; bamboos; food residues selected from bagasse, chaff, coffee beans, and molasses; and combinations of these raw materials.

8. The liquid mineral concentrate composition according to claim 6, wherein the activated carbon of a plant-derived raw material is palm shell activated carbon.

9. The liquid mineral concentrate composition according to any one of claims 1 to 8, having a pH of 7.5 to 10.5.

10. A water, food, or drink comprising the liquid mineral concentrate composition according to any one of claims 1 to 9.

11. The water, food, or drink according to 10, for use in the prevention or improvement of acidification in an organism.
